# EUROPEAN PATENT APPLICATION

(11) **EP 2 359 820 A1**
(43) Date of publication of application: **24.08.2011**
(21) Application number: 11250097.0
(22) Date of filing: 28.01.2011
(51) Int. Cl.: A61K 31/12, A61K 36/9066, A61P 25/28

(54) **Shunt delivery of curcumin for the treatment Alzneimer**

(30) Priority: 29.01.2010 US 696588
(71) Applicant: Codman & Shurtleff, Inc., Raynham, MA 02767 (US)
(72) Inventor: DiAmauro, Thomas M., Southborough, MA 01772 (US); Dextradeur, Alan, Franklin, MA 02038 (US); Lilienfeld, Sean, Sharon, MA 02067 (US); Venugopalan, Ramakrishna, Malvern PA 19355 (US)
(74) Representative: Carpmaels & Ransford

(57) **Abstract**

A method for reducing or preventing a human brain disorder relating to the presence of a pathogenic substance in cerebrospinal fluid by selecting a human for treatment as a patient and placing a proximal end of a first catheter, having at least a first lumen, in a first sub-dural location within the brain of the patient to establish open communication between the first lumen and cerebrospinal fluid of the patient. For an extended period of time, a curcumin agent selected from at least one of curcumin, a curcumin hybrid and a curcumin analog is delivered to the cerebrospinal fluid to interact with the pathogenic substance to attenuate its effect on the brain.

## Description

### CROSS REFERENCE TO RELATED APPLICATION

The present application is a continuation-in-part of pending application United States Serial No. 12/359713, filed January 26, 2009.

### BACKGROUND OF THE INVENTION

### 1. Field of the Invention

The invention relates to a method for delivering curcumin to a patient and more particularly to directing a curcumin agent intracranially to treat a human brain disorder such as Alzheimer's disease.

### 2. Description of the Related Art

Human brain tissue includes gray and white matter suspended in cerebrospinal fluid within the cranium and nourished by blood delivered through cerebral arteries. The gray matter has closely spaced cell bodies of neurons, such as in the cerebral cortex, and the underlying white matter contains densely packed axons that transmit signals to other neurons. Brain tissue has different densities and comprises approximately eighty percent of the intracranial content, with blood and cerebrospinal fluid each normally comprising approximately ten percent.

Cerebrospinal fluid is produced in several connected chambers known as ventricles and typically is renewed four to five times per day. Cerebrospinal fluid in a healthy human flows slowly and continuously through the ventricles, propelled by pulsations of the cerebral arteries, flows around the brain tissues and the spinal column, and then through small openings into the arachnoid membrane, which is the middle layer of the meninges surrounding the brain parenchyma and ventricles, where the fluid is finally reabsorbed into the bloodstream.

There are a number of brain disorders that arise from neurotoxins or other pathogenic substances which can accumulate in cerebrospinal fluid. For example, it has long been recognized that aggregation of the protein amyloid-beta, which can be found in cerebrospinal fluid, contributes to the degenerative condition known as Alzheimer's disease. Microscopic damage to brain tissue leads to atrophy and a general decline in brain function known as dementia.

U.S. Patent No. 5,980,480 by Rubenstein et al. describe a method and apparatus for treating adult-onset dementia due to Alzheimer's disease, based on the premise that dysfunction of normal bodily processes can lead to deleterious materials being retained within cerebrospinal fluid. A portion of a patient's cerebrospinal fluid is removed at a controlled rate to encourage the patient's body to produce replacement cerebrospinal fluid and thereby dilute the concentration of any deleterious materials within the cranium. The withdrawn fluid is returned to a bodily space such as the peritoneal cavity. In one embodiment, a dialysate chamber is provided, its walls being coated with antibodies specific to target agents within the withdrawn cerebrospinal fluid. Alternatively, antibodies are bound to beads, strands or other structures that can be periodically exchanged within the dialysate chamber through a dialysate port.

In U.S. Publication No. 2009/0131850, Mark Geiger discloses an implanted pump and filter system having a drug or enzyme to clean and filter cerebrospinal fluid. One lumen of a dual lumen catheter withdraws cerebrospinal fluid into a drug and filter reservoir and the other lumen returns the treated fluid to the subarachnoid space of the patient.

An occlusion resistant hydrocephalic shunt is disclosed in U.S. Patent No. 7,582,068 by Koullick et al. having a proximal end located in brain tissue and a distal end located within the patient external to the brain or located external to the patient altogether. The proximal end of the shunt carries an occlusion resistant or anti-occlusion agent in drug eluting regions where clotting or tissue growth tend to occlude the lumen of the shunt. A great number of possible anti-occlusion pharmaceutical agents are listed including curcumin.

Curcumin has been applied to endovascular stents to reduce restenosis, such as disclosed in U.S. Publication 2008/0241352 by Shalaby.

The phenolic molecule curcumin is also known as diferuloylmethane or (E,E)-1,7-bis (4-hydroxy-3-methoxyphenyl)-1,6-heptadiene-3,5,-dione. Curcumin may be derived from a natural source, the perennial herb Curcuma longa L., which is a member of the Zingiberaceae family. The spice turmeric is extracted from the rhizomes of Curcuma longa L. and has long been associated with traditional-medicine treatments used in Hindu and Chinese medicine. Turmeric was administered orally or topically in these traditional treatment methods.

Curcumin has been fed to and injected into the carotid artery of transgenic mice, as described by Yang et al. in "Curcumin Inhibits Formation of Amyloid Beta Oligomers and Fibrils, Binds Plaques, and Reduces Amyloid in Vivo", J. Biol. Chem. vol. 280, pp. 5892-5901 (2005). The article proposes conducting human clinical trials for curcumin to investigate whether curcumin may prevent or perhaps treat Alzheimer's disease.

It is therefore desirable to have a simpler, more rapid and more effective technique for reducing or eliminating pathogenic substances within human cerebrospinal fluid.

### SUMMARY OF THE INVENTION

An object of the present invention is to provide a curcumin agent directly to cerebrospinal fluid for an extended period of time to attenuate the effects of a pathogenic substance on the brain of a human.

Another object of the present invention is to treat dementia by delivering a curcumin agent into the brain.

A further object of the invention is to allow continuous long-term delivery of a curcumin agent to areas of the body where targeted neurotoxins exist or could potentially form and thereby reduce or eliminate Alzheimer's-type dementia.

This invention results from the realization that at least one of curcumin, a curcumin hybrid and a curcumin analog delivered to a sub-dural or sub-meningeal location within the brain is likely to reduce or eliminate neurotoxins and other pathogens such as amyloid-beta to treat or prevent occurrence of a brain disorder, especially Alzheimer-type dementia. The terms curcumin, curcumin hybrid and curcumin analog are defined below. The term sub-dural as used herein is intended to include all tissues, fluids and spaces underlying the outermost layer of the dura mater of the meninges, and therefore includes intra-dural sinuses such as the superior sagittal sinus, as well as gray matter, white matter and the ventricles. The term sub-meningeal as used herein is intended to include everything underlying the pia mater, which is the inner-most layer of the meninges; in other words, the term sub-meningeal excludes the arachnoid and dura mater membranes and any spaces or sinuses within them. The term brain parenchyma as used herein is intended to include gray matter, white matter and other essential parts of the brain providing its function.

This invention features a method by which a human brain disorder relating to the presence of a pathogenic substance in cerebrospinal fluid is reduced or prevented by selecting a human for treatment as a patient and placing a proximal end of a first catheter, having at least a first lumen, in a first sub-dural location within the brain of the patient to establish open communication between the first lumen and cerebrospinal fluid of the patient. For an extended period of time, a curcumin agent selected from at least one of curcumin, a curcumin hybrid and a curcumin analog is delivered to the cerebrospinal fluid to interact with the pathogenic substance to attenuate its effect on the brain.

In one embodiment, the patient is selected because he or she was diagnosed as having a brain disorder, preferably as having Alzheimer's disease for purposes of treatment according to the present invention. In a preferred embodiment, curcumin achieves a brain tissue concentration of at least 0.1 µM, more preferably at least 1 µM, more preferably at least 5 µM, more preferably at least 20 µM.

In some embodiments, the proximal end of the catheter is placed within a ventricle of the brain as the first sub-dural location, and cerebrospinal fluid intermixed with the curcurmin agent is returned directly to the first sub-dural location. In some other embodiments, the cerebrospinal fluid intermixed with the curcurmin agent is returned directly to another sub-dural location within the brain of the patient. The exposed cerebrospinal fluid is returned to the patient through a second lumen of the first catheter, or through a second lumen of a second catheter.

In a further embodiment, the curcumin agent is released from a reservoir connected to the first catheter. Preferably, the reservoir includes a removable canister carrying curcumin agent or is otherwise rechargeable so that additional curcumin agent can be added to extend the treatment period or increase the dosage of curcumin agent. In one embodiment, a proximal catheter connected to the reservoir transports cerebrospinal fluid intermixed with the curcumin agent to another location within the patient such as the peritoneal cavity. In another embodiment, the curcumin agent is released from a coating carried within at least a portion of the first catheter.

In yet another embodiment, the method further includes sampling cerebrospinal fluid at a sub-dural location that is different than the first sub-dural location and measuring the concentration of the curcumin agent in the cerebrospinal fluid to estimate its brain tissue concentration.

This invention may also be expressed as a system and method for reducing or preventing a human brain disorder relating to the presence of a pathogenic substance in cerebrospinal fluid by selecting a human for treatment as a patient and placing a proximal end of a first catheter, having at least a first lumen, in a first sub-dural location, preferably a sub-meningeal location, within the brain of the patient to establish open communication between the first lumen and cerebrospinal fluid of the patient. Cerebrospinal fluid is withdrawn through the first lumen and is exposed to a curcumin agent selected from at least one of curcumin, a curcumin hybrid and a curcumin analog to interact with the pathogenic substance to attenuate its effect on the brain. The exposed cerebrospinal fluid is returned to the patient so that the curcumin agent achieves a brain tissue concentration of at least 0.1 µM.

### BRIEF DESCRIPTION OF THE DRAWINGS

In what follows, preferred embodiments of the invention are explained in more detail with reference to the drawings, in which:
FIG. 1A is a perspective cross-sectional view of a patient showing a proximal catheter positioned
   in a ventricle within the brain of the patient according to one embodiment of the present invention;
FIG. 1B is a side cross-sectional view of a dual lumen Ommaya-type reservoir system for
   delivering a curcumin agent according to the present invention;
FIG. 1C is a perspective view of a delivery pump system according to another embodiment of
   the present invention; and
FIGS. 1D-22 and FIG. 29 are incorporated by reference from U.S. Application No. 12/359,713 filed January 26, 2009 and entitled "Methylene Blue-Curcumin Analog for the Treatment of Alzheimer's Disease".

### DETAILED DESCRIPTION OF THE PRESENTLY PREFERRED EMBODIMENTS

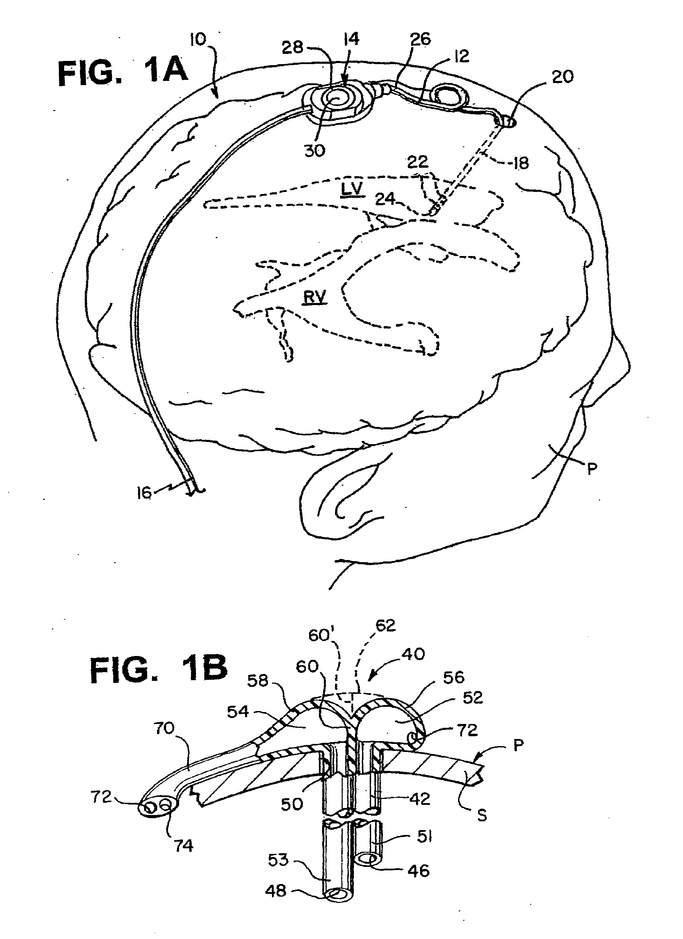

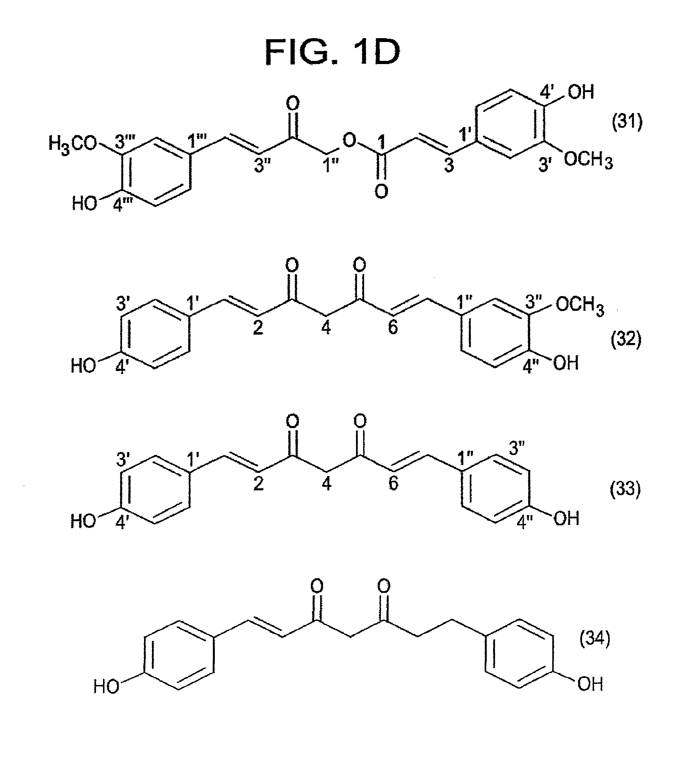

The term "curcumin agent" is described in more detail below. A portion of system 10 according to one aspect of the present invention is illustrated in FIG. 1A having a proximal catheter 12, a reservoir 14, and a distal catheter 16. A patient P is selected based on one or more criteria, such as having been diagnosed as having a brain disorder such as Alzheimer's disease. Reservoir 14 is implanted under the scalp of the patient P and a leading portion 18 of the proximal catheter 12, also referred to as a ventricular catheter in this construction, is placed through a burr hole 20 into a first sub-dural location such that inlet holes 22 of proximal end 24 communicate with cerebrospinal fluid in right ventricle RV. Alternatively, the proximal end 24 is placed in left ventricle LV or other sub-dural location, preferably sub-meningeal location, in communication with cerebrospinal fluid of patient P.

In this construction, catheter 12 defines at least a first lumen communicating with inlet holes 22 at proximal end 24 and with reservoir 14 at distal end 26. Cerebrospinal fluid is withdrawn through the first lumen and into reservoir 14 , which includes a removable canister 28 having access port 30. Preferably, curcumin agent is loaded into canister 28 prior to implantation of system 10, and then dosage amounts of curcumin agent can be extended or increased by needle injection through access port 30. Cannister 28 can also be removed and recharged or replaced as desired. Curcumin agent is delivered for an extended period of time to interact with one or more pathogenic substances to attenuate the effect of the pathogenic substance on the brain of the patient. Preferably, a desired brain tissue concentration of curcumin agent is achieved as described below.

Distal catheter 16 returns cerebrospinal fluid, exposed to curcumin agent according to the present invention, to another location within the patient P such as the peritoneal cavity. Alternatively, another lumen returns exposed cerebrospinal fluid to a sub-dural location such as shown in FIG. 1B for Ommaya-type system 40 according to another aspect of the present invention. First catheter 42 and second catheter 44 having lumens 46 and 48, respectively, are passed through burr hole 50 in skull S of patient P. In an alternative construction, lumens 46 and 48 are defined by a single catheter. Typically, proximal end 51 of first catheter 42 is placed in a first sub-dural location to withdraw cerebrospinal fluid and proximal end 53 is placed in a second sub-dural location, which can be the same or different space, tissue or region of the brain, to return exposed cerebrospinal fluid directly to the brain. In another construction, curcumin agent is simply delivered through lumen 48 into cerebrospinal fluid while other cerebrospinal fluid is withdrawn through lumen 46 for sampling and measuring purposes.

System 40 can be derived from or can utilize devices such as the Codman 3000 constant flow rate wound capillary tube device, with dosage of curcumin agent being increased by increasing its concentration as passed through the capillary tubes. Another device is the Archimedes device with etched chip having a tortuous path to define flow rate of delivered fluids. Both devices are currently commercially available from Codman & Shurtleff, Inc. of Raynham, Massachusetts.

As illustrated in FIG. 1B in partial side cross-sectional view, lumen 46 communicates with a first chamber 52 defined by dome wall 56 and lumen 48 communicates with a second chamber 54 defined by dome wall 58, which is separated from chamber 52 by septum 60. Dome walls 56 and 58 can be separately tactilely discerned and palpated to move fluid therethrough. In another construction as shown in phantom, septum 60 continues upward as septum 60' to a single dome wall 62 defining or covering both chambers 52 and 54. In either construction, a check valve can be provided to allow fluid to flow in only one direction between chambers 52 and 54 during palpation. A separate "squeeze-bulb"-type chamber can also be provided for mechanical or electro-mechanical activation as desired.

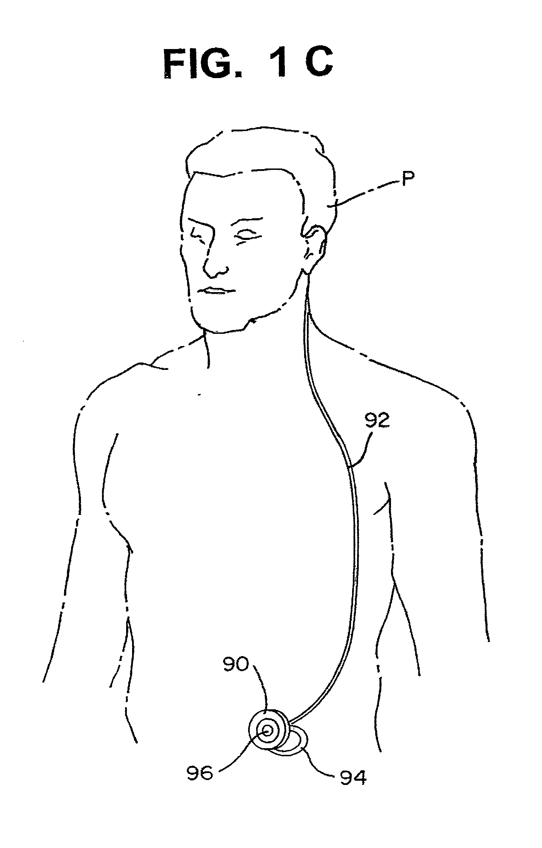

As further illustrated in FIG. 1B, system 40 in one construction includes multi-lumen catheter 70 defining a lumen 72 which communicates with chamber 52 and a lumen 74 which communicates with chamber 54 to enable fluid to be remotely withdrawn, delivered and/or returned separately and independently for each chamber 52, 54. A remote delivery pump 90 can be utilized such as shown in FIG. 1C to communicate with one or more lumens through catheter 92 implanted under the skin of patient P. One example of a suitable pump is the CODMAN Medstream pump with piezo-electric valve having a variable duty cycle which is currently commercially available from Codman & Shurtleff, Inc. of Raynham, Massachusetts. Other types of pumps or delivery devices can be utilized to provide curcumin agent directly to cerebrospinal fluid for days, weeks or months as desired according to the present invention.

Cerebrospinal fluid can be exposed to a curcumin agent according to the present invention in one or more reservoirs or by contact with a coating, a surface or a porous substrate containing curcumin agent. In one construction a hydrophilic, lipophobic membrane, preferably carried in a removable, replacable cartridge, traps a gel containing curcumin agent and allows only cerebrospinal fluid to flow therethrough. A minimum porosity can be provided to trap particles therein, such as bound amyloid-beta particles or other bound pathogenic substances. Alternatively, curcumin agent is compounded with catheter material such as silicone or is mechanically entrapped by a substrate such as one disclosed in U.S. Pub. No. 2006/0004317 by Mauge et al. entitled "Hydrocephalus Shunt" which is incorporated herein by reference. Some or all of the length of the catheter can be "doped" with curcumin agent so that it elutes directly into cerebrospinal fluid.

In constructions where catheter 92 does not define another lumen to return curcumin-exposed cerebrospinal fluid to a sub-dural location, the exposed cerebrospinal fluid is returned to another location within the patient by a catheter 94, such as to the peritoneal cavity. Samples of cerebrospinal fluid, before and/or after exposure to a curcumin agent, can be accomplished through port 96. Alternatively, bound pathogenic substances can be periodically withdrawn from the patient's cerebrospinal fluid and tested or discarded safely.

The term "curcumin agent" as used herein includes "curcumin", a "curcumin hybrid", and/or a "curcumin analog" as defined below and in a priority application entitled "Methylene Blue-Curcumin Analog for the Treatment of Alzheimer's Disease", U.S. Application No. 12/359,713 filed January 26, 2009 which is incorporated herein by reference in its entirety. Commercial curcumin typically includes three major components: curcumin (77%), demethoxycurcumin (17%), and bisdemethoxycurcumin (3%), which are often referred to as "curcuminoids." As used herein, the term "curcumin" includes any one or more of these three major components of commercial curcumin, and any active derivative of these agents. This includes natural and synthetic derivatives of curcumin and curcuminoids, and includes any combination of more than one curcumenoid or derivative of curcumin. Derivatives of curcumin and curcumenoids include those derivatives disclosed in U.S. Patent Application Publication 20020019382, which is herein specifically incorporated by reference.

Modifications of curcumin and its functional fragments that either enhance or do not greatly affect the ability to treat Alzheimer's disease are also included within the term "curcumin." Such modifications include, for example, additions, deletions or replacements of one or more functional groups. These modifications will either enhance or not significantly alter the structure, conformation or functional activity of curcumin or a functional fragment thereof. Additionally, curcumin or its functional fragments can be modified by the addition of epitope tags or other sequences that aid in its purification and which do not greatly affect its activity. As used herein, the term "functional fragment," in connection with an curcumin, is intended to mean any portion of curcumin that maintains its to inhibit oxidiation, or to prevent amyloid-beta oligomer formation. If desired, a functional fragment can include regions of the curcumin with activities that beneficially cooperate with the ability to inhibit oxidation or oligomer formation.

Curcumin is soluble in ethanol, alkalis, ketones, acetic acid and chloroform. It is insoluble in water. Curcumin is therefore lipophilic, and generally readily associates with lipids. In certain embodiments, curcumin can also be formulated as a metal chelate.

Also in accordance with the present invention, publicly known analogs of curcumin may be used. As used herein, "curcumin analogs" are those compounds which due to their structural similarity to curcumin, exhibit anti-proliferative or pro-apoptotic effects on cancer cells similar to that of curcumin. Curcumin analogs which may have anti-cancer effects similar to curcumin include Ar-tumerone, methylcurcumin, demethoxy curcumin, bisdemethoxycurcumin, sodium curcuminate, dibenzoylmethane, acetylcurcumin, feruloyl methane, tetrahydrocurcumin, 1,7-bis(4-hydroxy-3-methoxyphenyl)-1,6-heptadiene-3,5-dione (curcumin1), 1,7-bis(piperonyl)-1,6-heptadiene-3,5-dione(piperonyl curcumin)1,7-bis(2-hydroxy naphthyl)-1,6-heptadiene-2,5-dione(2-hydroxyl naphthyl curcumin), 1,1-bis(phenyl)-1,3,8,10-undecatetraene-5,7-dione (cinnamyl curcumin) and the like (Araujo and Leon, 2001; Lin et al., 2001; John et al., 2002; see also Ishida et al., 2002). Curcumin analogs may also include isomers of curcumin, such as the (Z,E) and (Z,Z) isomers of curcumin. In a related embodiment, curcumin metabolites which have anti-cancer effects similar to curcumin can also be used in the present invention. Known curcumin metabolites include glucoronides of tetrahydrocurcumin and hexahydrocurcumin, and dihydroferulic acid. In certain embodiments, curcumin analogs or metabolites can be formulated as metal chelates, especially copper chelates. Other appropriate derivatives of curcumin, curcumin analogs and curcumin metabolites appropriate for use in the present invention will be apparent to one of skill in the art.

In some embodiments, the curcumin analogs are those found in US Published patent application US 2005/0181036. In other embodiments, the curcumin analogs are those found in US Published patent application US 2006/0067998. In yet other embodiments, the curcumin analogs are those found in US Published patent application US 2005/0267221.

In certain aspects, 1,7,-bis(4-hydroxy-3-methoxyphenyl)-1,6-heptadi- ene-3,5-dione is the curcumin that may be used in the present invention. Other curcumin analogs (curcuminoids) that may be used include, for example, demethoxycurcumin, bisdemethoxycurcumin, dihydrocurcumin, tetrahydrocurcumin, hexahydrocurcumin, dihydroxytetrahydrocurcumin, Yakuchinone A and Yakuchinone B, and their salts, oxidants, reductants, glycosides and esters thereof. Such analogs are described in U.S. Patent Application 20030147979; and U.S. Pat. No. 5,891,924 both of which are incorporated in their entirety herein by reference.

Other curcumin analogs (curcuminoids) that may be used according to the present invention include dihydroxycurcumin and NDGA. Further examples of curcumin analogs include but are not limited to (a) ferulic acid, (i.e., 4-hydroxy-3-methoxycinnamic acid; 3,4-methylenedioxy cinnamic acid; and 3,4-dimethoxycinnamic acid); (b) aromatic ketones (i.e., 4-(4-hydroxy-3-methoxyphenyl)-3-buten-2-one; zingerone; -4-(3,4-methylenedioxyphenyly-2-butanone; 4-(p-hydroxyphenyl)-3-buten-2-one; 4-hydroxyvalerophenone; 4-hydroxybenzylactone; 4-hydroxybenzophenone; 1,5-bis(4-dimethylaminophen- yl)-1,4-pentadien-3-one); (c) aromatic diketones (i.e., 6-hydroxydibenzoylmethane) (d) caffeic acid compounds (i.e., 3,4-dihydroxycinnamic acid); (e) cinnamic acid; (f) aromatic carboxylic acids (i.e., 3,4-dihydroxyhydrocinnainic acid; 2-hydroxycinnamic acid; 3-hydroxycinnamic acid and 4-hydroxycinnamic acid); (g) aromatic ketocarboxylic acids (i.e., 4-hydroxyphenylpyruvic acid); and (h) aromatic alcohols (i.e., 4-hydroxyphenethyl alcohol). These analogs and other representative analogs that can be used in the present invention are further described in WO9518606 and WO01040188, which are incorporated herein by reference in their entirety.

Curcumin or analogs thereof may be purified from plants or chemically synthesized using methods well known and used by those of skill in the art. Plant-derived curcumin and/or its analogs can be obtained by extraction from plants including Zingiberaceae Curcuma, such as Curcuma longa (turmeric), Curcuma aromatica (wild turmeric), Curcuma zedoaria (zedoary), Curcuma xanthorrhiza, mango ginger, Indonesian arrowroot, yellow zedoary, black zedoary and galangal. Methods for isolating curcuminoids from turmeric are well known in the art (Janaki and Bose, 1967). Still further, curcumin may be obtained from commercial sources, for example, curcumin can be obtained from Sigma Chemicals Co (St. Louis, Mo.).

Any conventional method can be used to prepare curcumin and its analogs to be used in the present invention. For example, turmericoleoresin, a food additive, which essentially contains curcumin, can be produced by extracting from a dry product of rhizome of turmeric with ethanol at an elevated temperature, with hot oil and fat or propylene glycol, or with hexane or acetone at from room temperature to a high temperature. Alternatively, those can be produced by the methods disclosed in Japanese Patent Applications 2000-236843, H-11-235192 and H-6-9479, and U.S. Patent Application No. 20030147979, which is incorporated by reference herein in its entirety.

In certain embodiments, a purified product of at least one curcumin and/or its analog may be delivered to cerebrospinal fluid according to the present invention. Alternatively, a semi-purified or crude product thereof may be used, provided that it does not contain impurities which may not be acceptable as a pharmaceutical or food product.

There has been limited testing of the potency of curcumin analogs against amyloid-beta. Park, J. Nat. Prod., 65,9, Sept. 2002, reports testing the following curcumin analogs for the ability to provide in vitro protection for PC12 cells against amyloid-beta insult (with reference numbers 31-34 from FIG. ld of U.S. Appln. 12.359,713 which has been incorporated herein by reference:
4"-(3"'-methoxy-4"'-hydroxyphenyl)-2"-oxo-3"-enebutanyl3-(3'-methoxy-4'hydroxyphenyl) propenoate (31);
1-(4-hydroxy-3-methoxyphenyl)-7-(4-hydroxyphenyl)-1,6-heptadiene-3,5-dione (demethoxycurcumin)(32);
1,7-bis(4-hydroxyphenyl)-1,6-heptadiene-3,5-dione (bisdemethoxycurcumin), (33); and
1,7 -bis(4-hydroxyphenyl)-1-heptene-3,5-dione (34).

Analysis of the Park data reveals that each of compounds (31)-(34) is a more potent neuroprotectant against amyloid-beta than curcumin, with compounds (31) and (34) being on the order of 5 and 10 fold more potent. Therefore, in preferred embodiments, each of compounds (31)-(34) is used by itself or in combination as the parent compound for the manufacturing and use of a curcumin prodrug. Each of the parent compounds may be obtained by the methods disclosed in Park.

In other embodiments, the curcumin is combined with a second lipophilic therapeutic agent, preferably another polyphenol such as resveratrol, to form a "curcumin hybrid" which is delivered to cerebrospinal fluid according to the present invention. In some embodiments, the curcumin is provided in a formulation with another compound selected from the group consisting of gingko biloba extract, resveratrol, hispidin, genistein, ellagic acid, 1,25 dihydroxyvitamin D3, the green tea catechin EGCG, and docosahexaenoic acid (DHA).

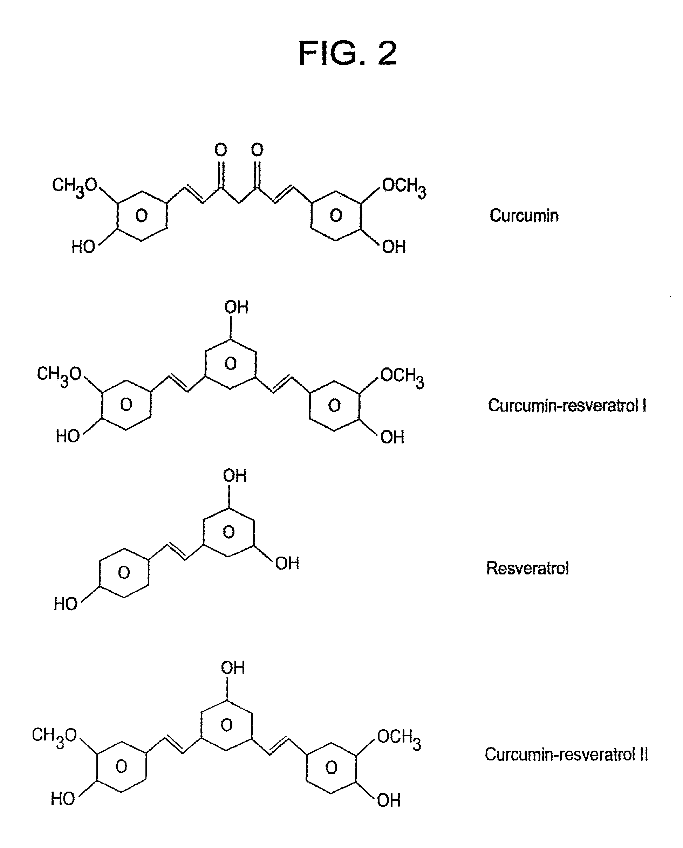

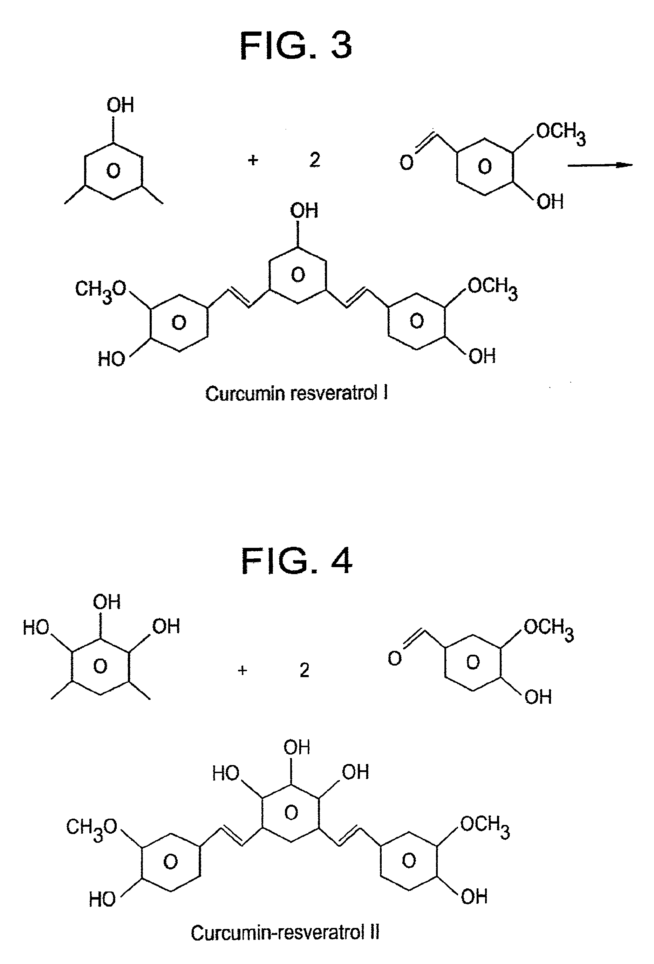

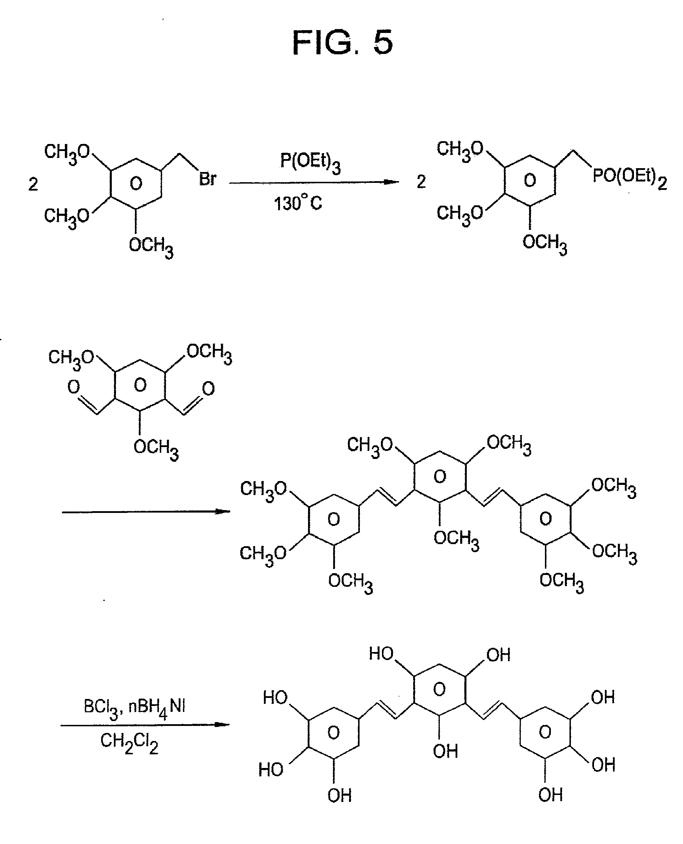

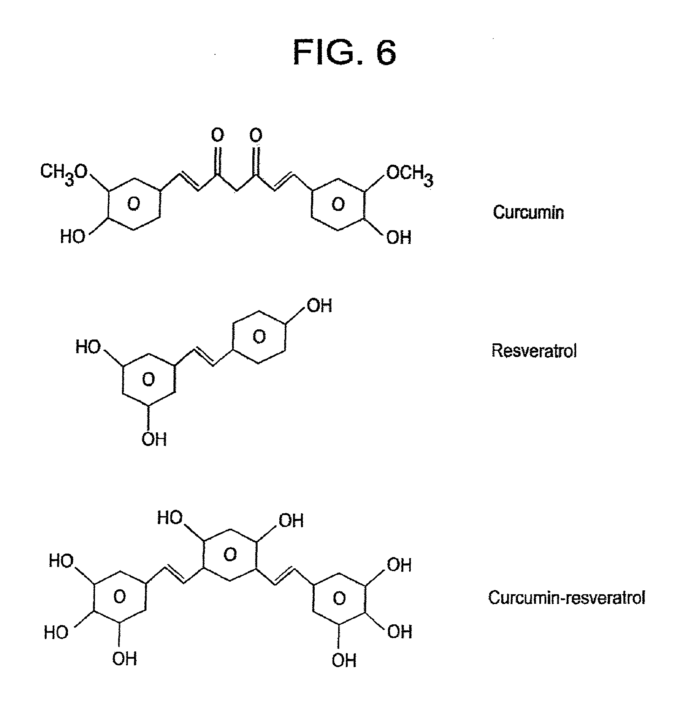

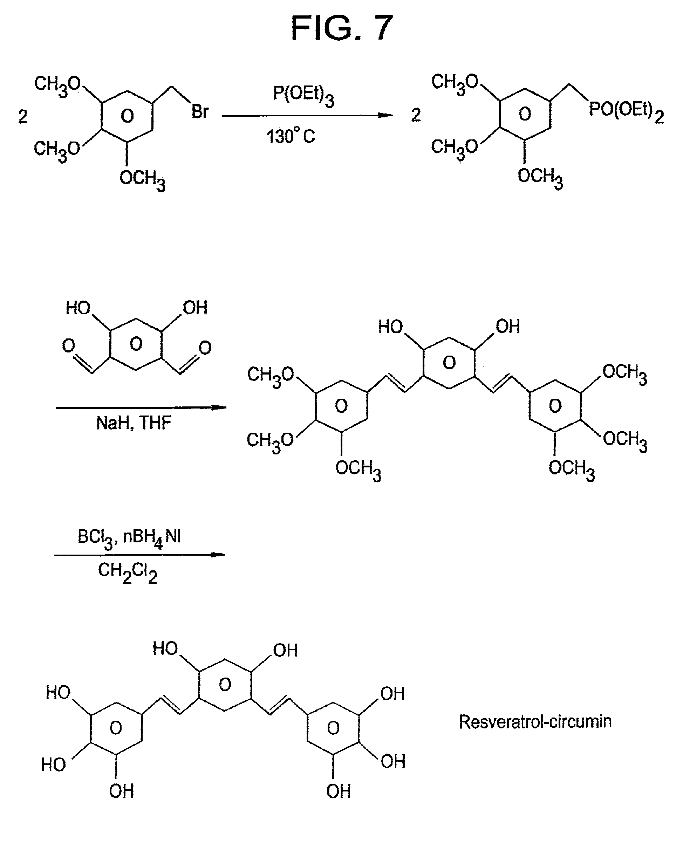

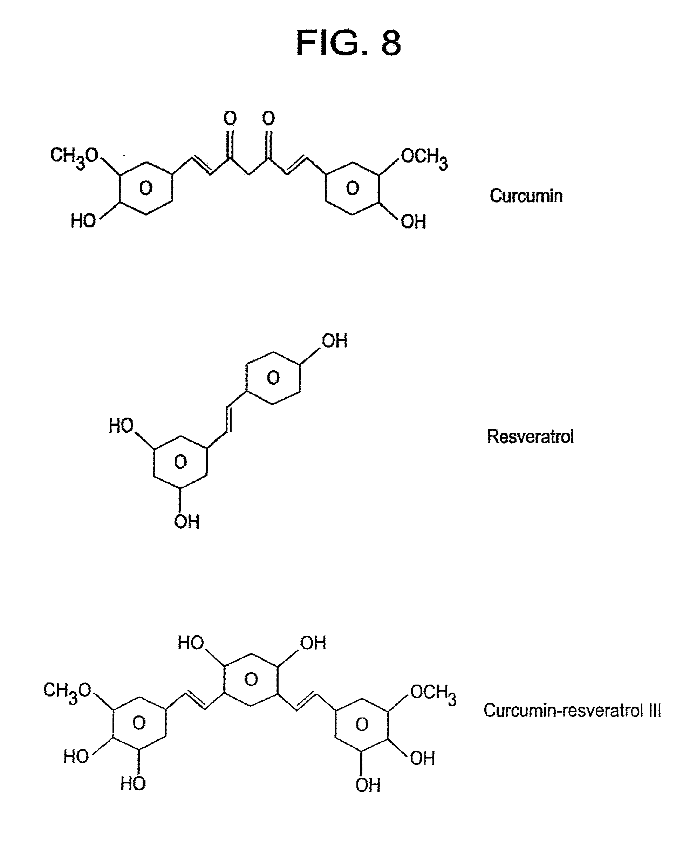

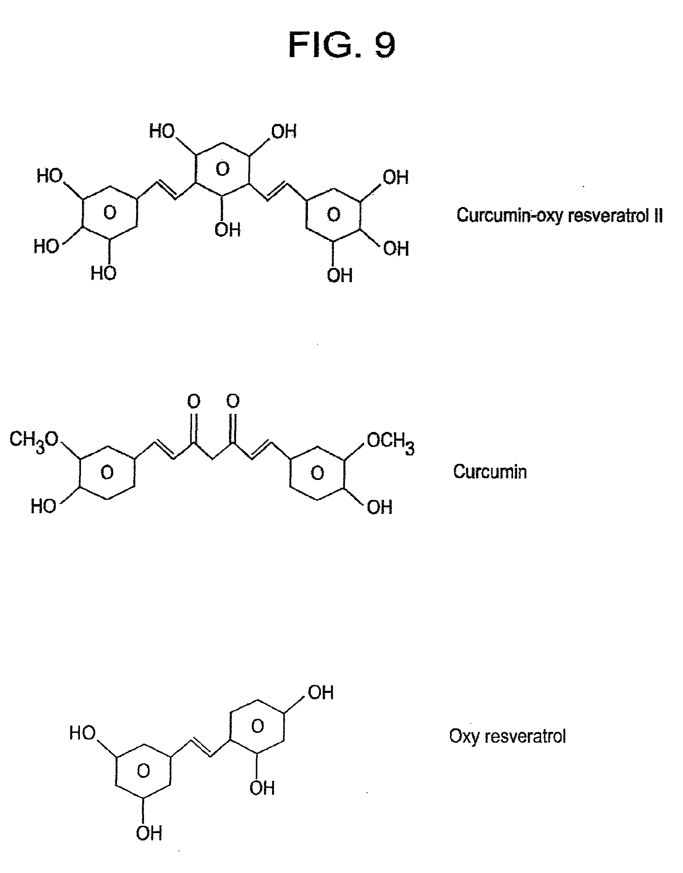

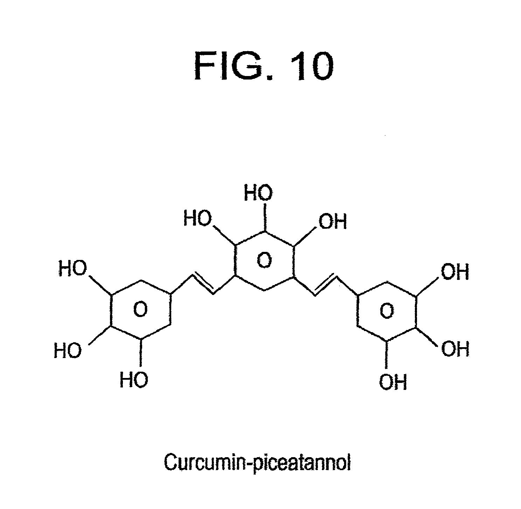

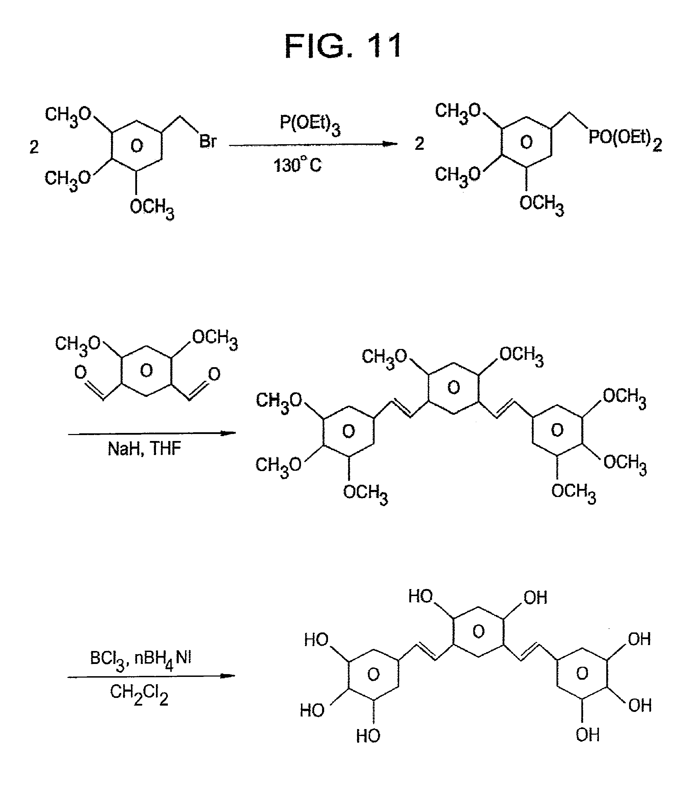

There are a number of curcumin hybrids as well as analogs described in U.S. Appln. No. 12/359,713, which has been incorporated herein by reference, to be delivered to cerebrospinal fluid according to the present invention. Figures 2-16 of U.S. Appln. No. 12/359,713 disclose various curcumin derivatives that are hybrids of curcumin and various other natural polyphenols. Each of these derivatives is a triphenolic compound, wherein the intermediate diketone structure of curcumin is replaced with a phenolic group. The resulting compound retains the spacing between the two phenols of curcumin, and also possesses the biphenolic spacing of the additional polyphenol. FIG. 2 of U.S. Appln. No. 12/359,713 discloses the structures of curcumin, resveratrol, and two curcumin-resveratrol hybrids. Each of the hybrids retains the interphenolic spacing of each of curcumin and reveratrol. FIG. 3 of U.S. Appln. No. 12/359,713 discloses a method of making the curcumin-resveratrol I hybrid. FIG. 4 of U.S. Appln. No. 12/359,713 discloses a method of making the curcumin-resveratrol II hybrid. FIG. 5 of U.S. Appln. No. 12/359,713 discloses a method of making a curcumin-resveratrol hybrid having three hydroxyl groups in each of the central phenolic group and lateral phenolic groups. FIG. 6 of U.S. Appln. No. 12/359,713 discloses curcumin, resveratrol and a hybrid thereof, wherein all of the phenolics of the natural compounds are represented in the hybrid, providing trihydroxyl lateral phenolic groups and a dihydroxyl central phenolic group. FIG. 7 of U.S. Appln. No. 12/359,713 discloses a method of making the curcumin-resveratrol hybrid of FIG. 6. FIG. 8 of U.S. Appln. No. 12/359, 713 is similar to the hybrid of FIG. 6, but wherein the methoxy groups of the base curcumin molecule are retained. FIG. 9 of U.S. Appln. No. 12/359,713 discloses curcumin, oxyresveratrol and a hybrid thereof, wherein all of the hydroxyls/phenolics of the natural compounds are represented in the hybrid, providing trihydroxyl lateral phenolic groups and a trihydroxyl central phenolic group. FIG. 10 of U.S. Appln. No. 12/359,713 discloses curcumin, piceatannol and a hybrid thereof, wherein all of the hydroxyls/phenolics of the natural compounds are represented in the hybrid, providing trihydroxyl lateral phenolic groups and a trihydroxyl central phenolic group. FIG. 11 of U.S. Appln. No. 12/359,713 discloses a method of making a curcumin-resveratrol hybrid,wherein all of the hydroxyls/phenolics of the natural compounds are represented in the hybrid, providing trihydroxyl lateral phenolic groups and a dihydroxyl central phenolic group.

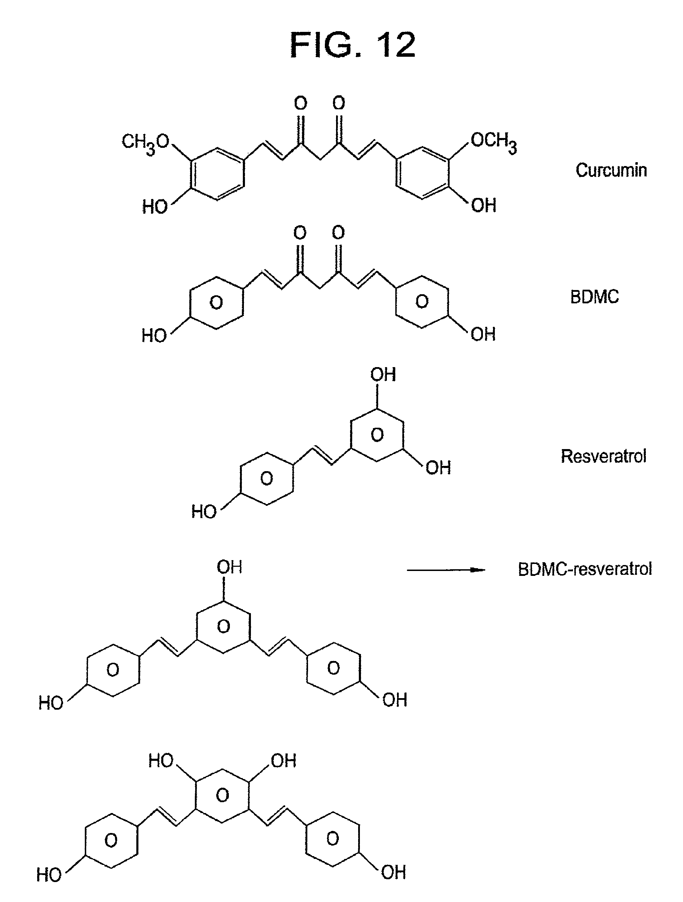

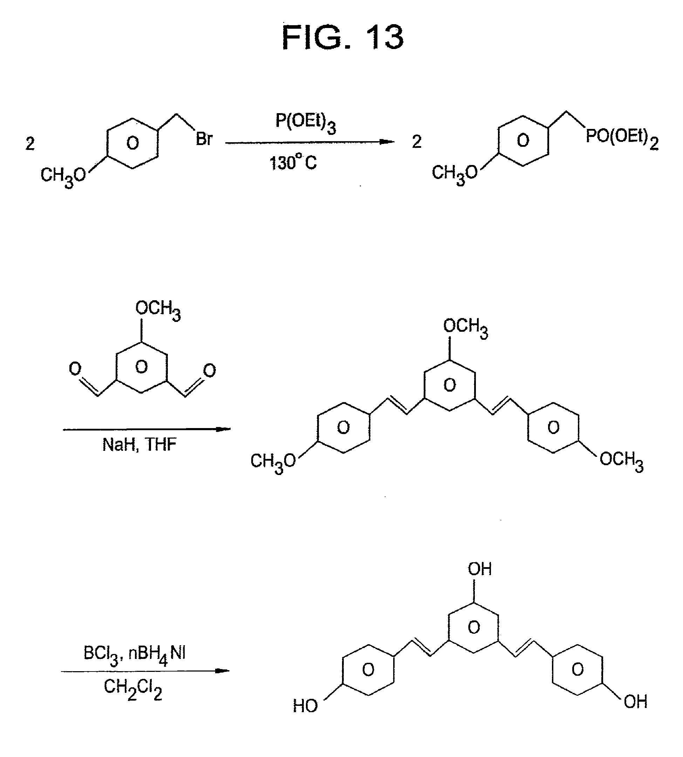

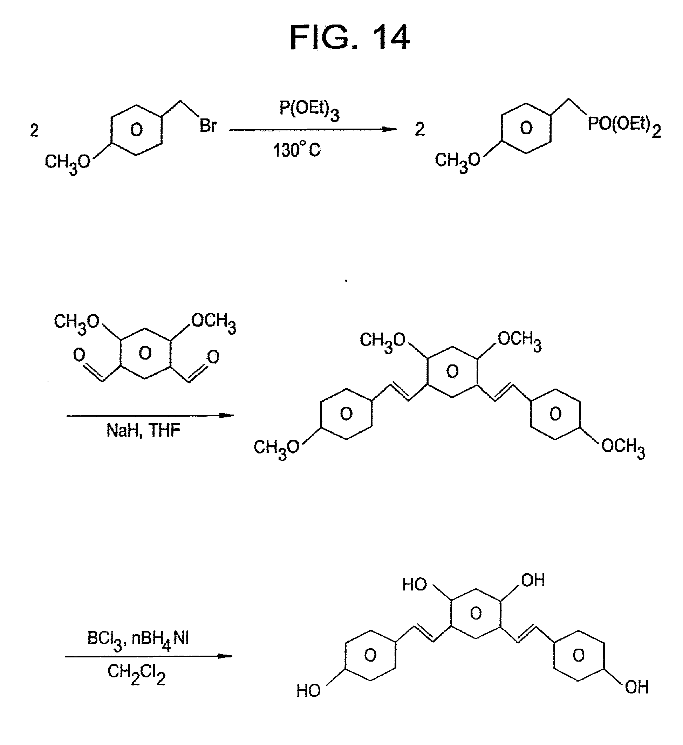

FIG.12 of U.S. Appln. No. 12/359,713, incorporated herein by reference, discloses curcumin, BDMC, resveratrol and curcumin hybrids thereof, wherein all of the phenolics of the natural compounds are represented in the hybrid, providing hydroxyl demethoxy lateral phenolic groups and a hydroxy or dihydroxyl central phenolic group. FIG. 13 of U.S. Appln. No. 12/359,713 provides a method of making the compound of FIG. 12 that has hydroxyl demethoxy lateral phenolic groups and a hydroxy central phenolic group. The curcumin analog molecule shown in FIG. 13 is 1-hydroxyl 3,5-bis (4'-hydroxyl styryl) benzene. Through simple deletion of a methoxy group in one of the reactants, 3,5-bis (4'-hydroxyl styryl) benzene can be made via method substantially similar to that shown in FIG. 13. FIG. 14 of U.S. Appln. No. 12/359,713 provides a method of making the compound of FIG. 12 that has hydroxyl demethoxy lateral phenolic groups and a dihydroxy central phenolic group. The curcumin analog molecule shown in FIG. 14 is 1,3-dihydroxyl 4,6-bis (4'-hydroxyl styryl) benzene.

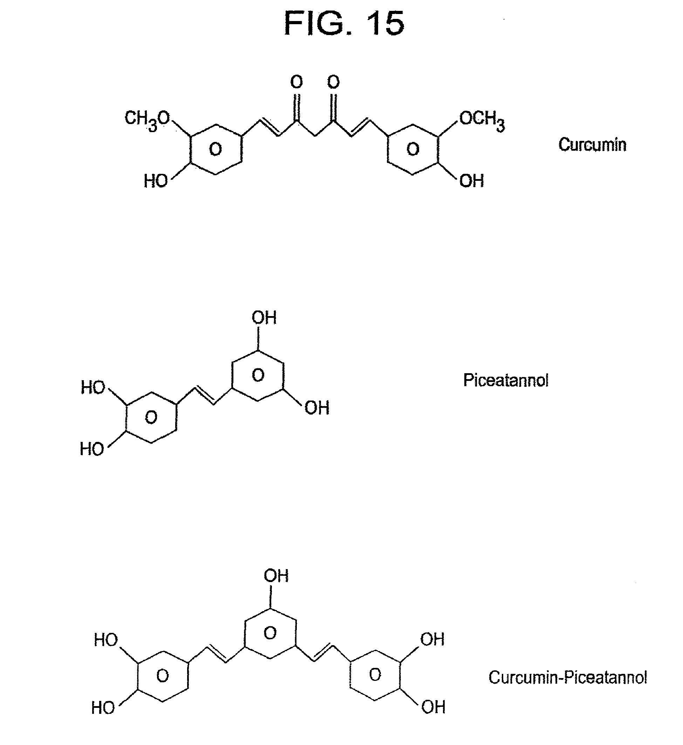

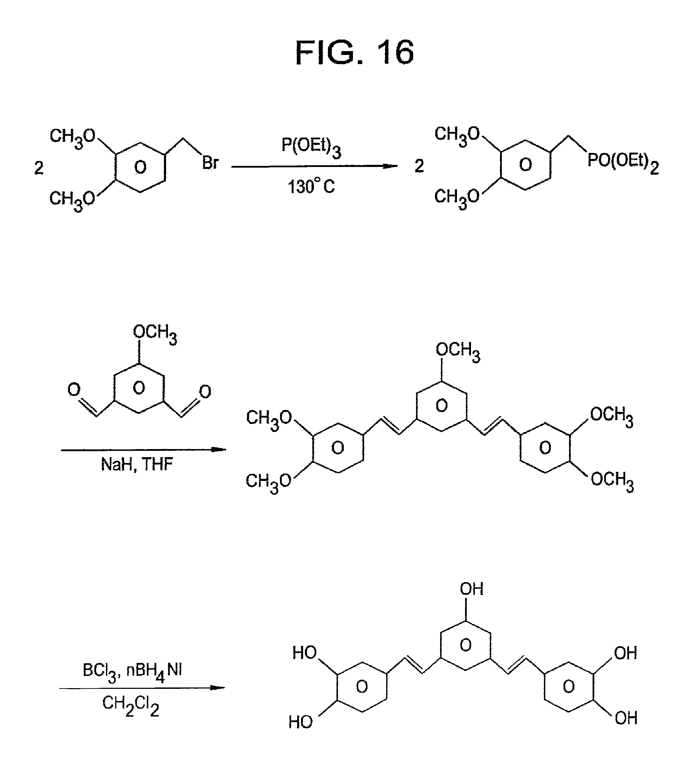

Figure 15 of U.S. Appln. No. 12/359,713 discloses curcumin, piceatannol and a hybrid thereof, wherein most of the hydroxyls of the natural compounds are represented in the hybrid, providing dihydroxyls in the end phenolic groups and a single hydroxyl in the central phenolic group in the positions common with the two natural compounds. Kim, Ann N Y Acad Sci., 2007 Jan;1095:473-82 reports that piceatannol treatment attenuates the intracellular accumulation of ROS induced by treatment of PC12 cells with Aβ, and inhibited Aβ-induced apoptotic features including internucleosomal DNA fragmentation, nucleus condensation, cleavage of poly(ADP-ribose) polymerase (PARP), and activation of caspase-3. The curcumin-piceatannol analog molecule shown in FIG. 15 is 1-hydroxyl 3,5-bis (3', 4'-dihydroxyl styryl) benzene. Through simple deletion of a methoxy group in one of the reactants, 3,5-bis (3', 4'-dihydroxyl styryl) benzene. can be made via method substantially similar to that shown in FIG. 15. FIG. 16 of U.S. Appln. No. 12/359,713 provides a method of making the compound of FIG. 15.

In some embodiments, a curcumin analog is utilized comprising at least one structure selected from the group consisting of:
a) 1-hydroxyl 3,5-bis (4'-hydroxyl styryl) benzene,
b) 1,3-dihydroxyl 4,6-bis (4'-hydroxyl styryl) benzene,
c) 1-hydroxyl 3,5-bis (3', 4'-dihydroxyl styryl) benzene, and
d) 3,5-bis (3', 4'-dihydroxyl styryl) benzene.
In some embodiments, a curcumin analog is utilized comprising 1-hydroxyl 3,5-bis (4'-hydroxyl styryl) benzene. In some embodiments, there is provided a curcumin analog comprising 3,5-bis (4'-hydroxyl styryl) benzene. In some embodiments, there is provided a curcumin analog comprising 1,3-dihydroxyl 4,6-bis (4'-hydroxyl styryl) benzene. In some embodiments, there is provided a curcumin analog comprising 1-hydroxyl 3,5-bis (3'. 4'-dihydroxyl styryl) benzene. In some embodiments, there is provided a curcumin analog comprising 3,5-bis (3', 4'-dihydroxyl styryl) benzene.

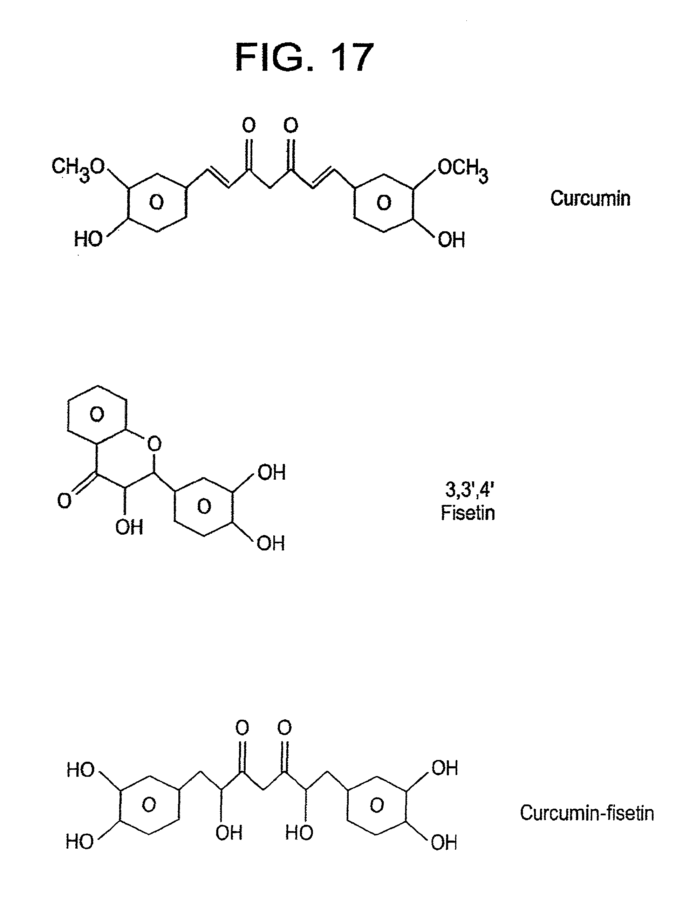

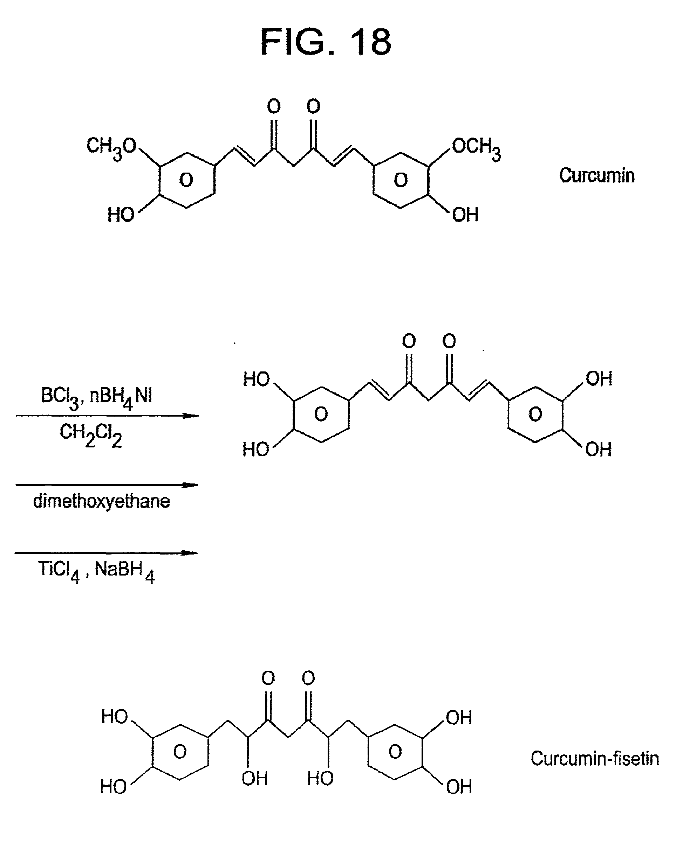

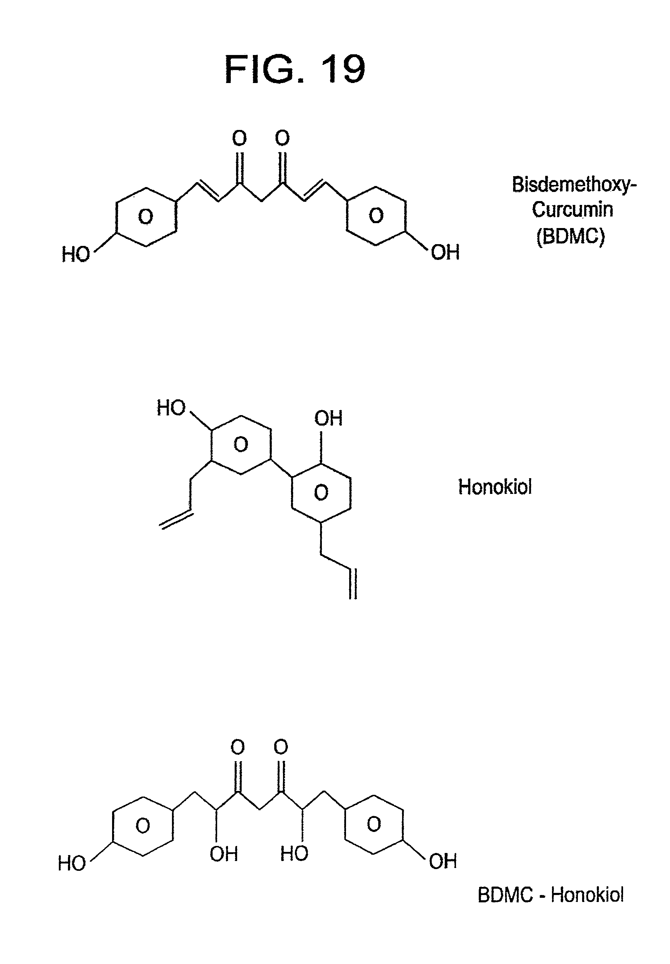

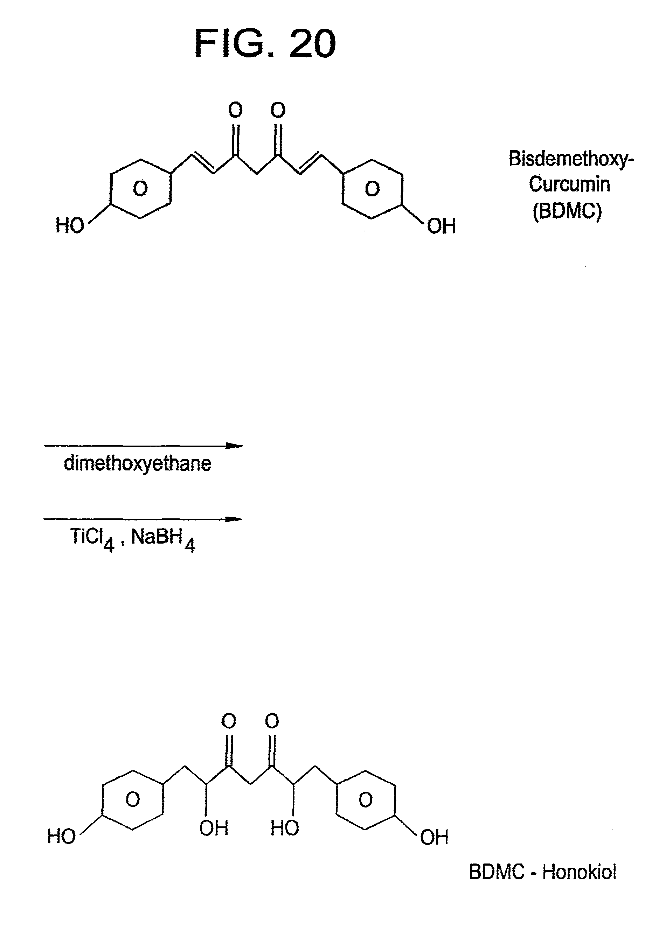

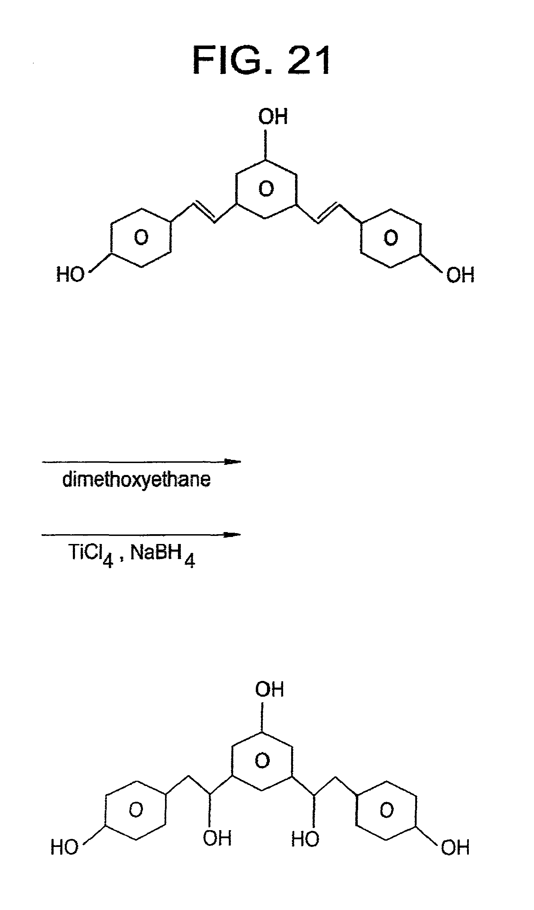

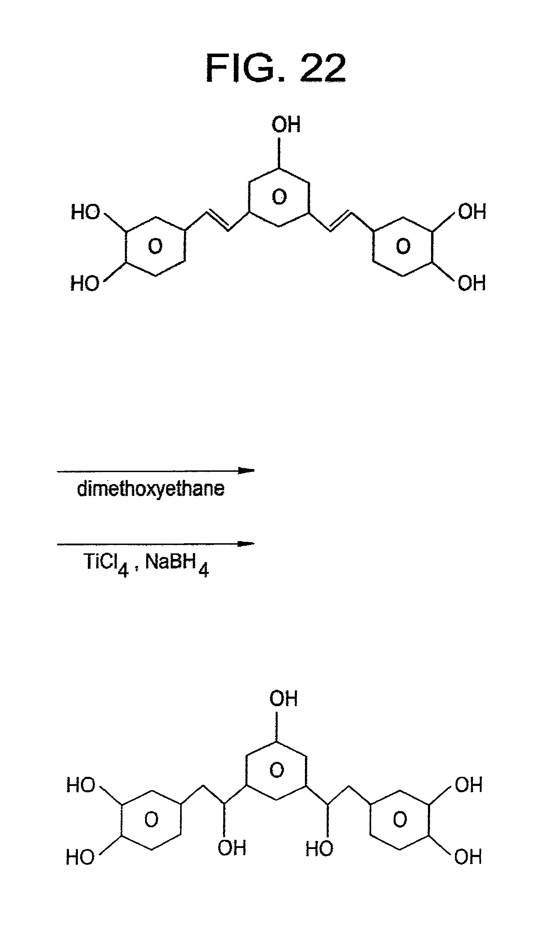

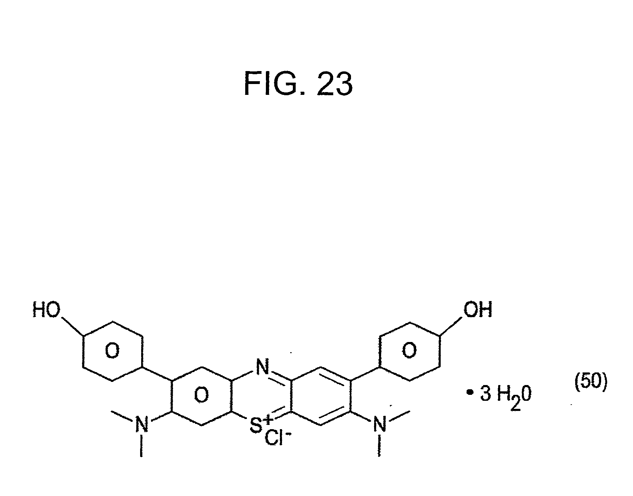

Further curcumin hybrids delivered to cerebrospinal fluid according to the present invention are disclosed in Figures 17-22 of U.S. Appln. No. 12/359,713 and are incorporated herein by reference. FIG. 17 of U.S. Appln. No. 12/359,713 discloses the structures of curcumin, 3,3',4' fisetin and a curcumin- 3,3',4' fisetin hybrid, wherein all of the hydroxyls of the curcumin and 3,3',4' fisetin compounds are represented in the hybrid, providing dihydroxyls in the end phenolic groups and a hydroxyl in the place of each double bond. Maher, Free Radic Res. 2006 Oct;40(10):1105-11 reports that fisetins in general and 3,3',4' fisetin in particular have potent (low micromolar) neurotrophic properties. FIG. 18 of U.S. Appln. No. 12/359,713 discloses a method of making the curcumin-3,3',4' fisetin hybrid of FIG. 17. FIG. 19 of U.S. Appln. No. 12/359,713 discloses the structures of curcumin, honokiol and a curcumin- honokiol hybrid, wherein all of the hydroxyls of the curcumin and honokiol compounds are represented in the hybrid, providing a single hydroxyl in the end phenolic groups and a hydroxyl in the place of each double bond. Fukuyama, Bioorg Med Chem Lett. 2002 Apr 22;12(8):1163-6 reports that honokiol has potent neurotrophic properties. FIG. 20 of U.S. Appln. No. 12/359,713 discloses a method of making the curcumin- honokiol hybrid of FIG. 19. FIG. 21 of U.S. Appln. No. 12/359,713 discloses a method of making a FIG. 13 - honokiol hybrid, wherein all of the hydroxyls of the natural compounds are represented in the hybrid, providing single hydroxyl in the end phenolic groups in the positions common with the two natural compounds, a hydroxyl in the central phenolic group, and a hydroxyl in the place of each curcumin double bond. FIG. 22 of U.S. Appln. No. 12/359,713 discloses a method of making a FIG. 15 - 3,3',4' fisetin hybrid, wherein all of the phenolics of the natural compounds are represented in the hybrid, providing single hydroxyl in the end phenolic groups and a hydroxy central phenolic group in the positions common with the two natural compounds, and an additional hydroxyl in the place of each curcumin double bond.

Another curcumin hybrid delivered to cerebrospinal fluid according to the present invention includes methylene blue as disclosed in U.S. Appln. No. 12/359,713 and incorporated herein by reference. As noted in U.S. Appln. No. 12/359,713, the seven-carbon hydrophobic medial section of curcumin (or BDMC) has the same length as the nonpolar tricyclic medial section of methylene blue. Moreover, the seven-carbon hydrophobic medial section of curcumin (or BDMC) provides resonance through spaced double bonds in a manner likely to be functionally similar to the double bonds of the nonpolar tricyclic section of methylene blue. Accordingly, it is believed that the pair of lateral hydroxyphenyl groups of curcumin (or BDMC) can be added onto methylene blue in a manner that preserves both their spatial relationship (and therefore their amyloid binding ability) and their interaction with the medial double bonds of curcumin. The result thereof is a molecule (50) having many of the desirable qualities of both curcumin and methylene blue. This molecule (50) is shown in FIG. 29 U.S. Appln. No. 12/359,713 and is named 2,6-bis (4', 4" dihydroxyphenyl), 3.7-bis (dimethylamino)-phenazathionium chloride. Therefore, in another embodiment of the present invention, there is provided a hybrid of curcumin (or BDMC) and methylene blue which is delivered to cerebrospinal fluid according to the present invention. This hybrid of BDMC and methylene blue is shown as molecule (50) in FIG. 29 of U.S. Appln. No. 12/359,713.

Thus, while there have been shown, described, and pointed out fundamental novel features of the invention as applied to preferred embodiments thereof, it will be understood that various omissions, substitutions, and changes in the form and details of the devices illustrated, and in their operation, may be made by those skilled in the art without departing from the spirit and scope of the invention. For example, it is expressly intended that all combinations of those elements and/or steps that perform substantially the same function, in substantially the same way, to achieve the same results be within the scope of the invention. Substitutions of elements from one described embodiment to another are also fully intended and contemplated. It is also to be understood that the drawings are not necessarily drawn to scale, but that they are merely conceptual in nature. It is the intention, therefore, to be limited only as indicated by the scope of the claims appended hereto.

Every issued patent, pending patent application, publication, journal article, book or any other reference cited herein is each incorporated by reference in their entirety.

## Claims

1. A method for reducing or preventing a human brain disorder relating to the presence of a pathogenic substance in cerebrospinal fluid, comprising:
selecting a human for treatment as a patient;
placing a proximal end of a first catheter, having at least a first lumen, in a first sub-dural location within the brain of the patient to establish open communication between the first lumen and cerebrospinal fluid of the patient; and
delivering to the cerebrospinal fluid for an extended period of time a curcumin agent selected from at least one of curcumin, a curcumin hybrid and a curcumin analog to interact with the pathogenic substance to attenuate its effect on the brain.

2. The method as claimed in claim 1 wherein the patient has been diagnosed as having a brain disorder.

3. The method as claimed in claim 1 wherein the patient has been diagnosed as having Alzheimer's disease.

4. The method as claimed in any one of claims 1 to 3 wherein the proximal end of the catheter is placed within a ventricle of the brain as the first sub-dural location.

5. The method as claimed in any one of claims 1 to 4 wherein cerebrospinal fluid intermixed with the curcumin agent is returned directly to the first sub-dural location.

6. The method as claimed in any one of claims 1 to 4 wherein cerebrospinal fluid intermixed with the curcumin agent is returned directly to another sub-dural location within the brain of the patient.

7. The method as claimed in any one of claims 1 to 6 wherein the curcumin agent achieves a brain tissue concentration of at least 0.1 µM.

8. The method as claimed in any one of claims 1 to 6 wherein the curcumin agent achieves a brain tissue concentration of at least 1 µM.

9. The method as claimed in any one of claims 1 to 6 wherein the curcumin agent achieves a brain tissue concentration of at least 5 µM.

10. The method as claimed in any one of claims 1 to 6 wherein the curcumin agent achieves a brain tissue concentration of at least 20 µM.

11. The method as claimed in any one of claims 1 to 10 wherein the curcumin agent is released from a reservoir connected to the first catheter.

12. The method as claimed in claim 11 wherein a proximal catheter connected to the reservoir transports cerebrospinal fluid intermixed with the curcumin agent to another location within the patient.

13. The method as claimed in claim 11 wherein the reservoir includes a removable canister carrying curcumin agent.

14. The method as claimed in any one of claims 1 to 13 wherein at least some of the curcumin agent is released from a coating carried within at least a portion of the first catheter.

15. The method as claimed in any one of claims 1 to 14 further including sampling cerebrospinal fluid at a sub-dural location that is different than the first sub-dural location and measuring the concentration of the curcumin agent in the cerebrospinal fluid.

16. A method for reducing or preventing a human brain disorder relating to the presence of a pathogenic substance in cerebrospinal fluid, comprising:
selecting a human for treatment as a patient;
placing a proximal end of a first catheter, having at least a first lumen, in a first sub-dural location within the brain of the patient to establish open communication between the first lumen and cerebrospinal fluid;
withdrawing cerebrospinal fluid through the first lumen and exposing it to a curcumin agent including at least one of curcumin, a curcumin hybrid and a curcumin analog to interact with the pathogenic substance to attenuate its effect on the brain; and
returning the exposed cerebrospinal fluid to the patient, wherein the curcumin agent achieves a brain tissue concentration of at least 0.1 µM.

17. The method as claimed in claim 16 wherein the exposed cerebrospinal fluid is returned to the patient through a second lumen of the first catheter.

18. The method as claimed in claim 16 wherein the exposed cerebrospinal fluid is returned to the patient through a lumen of a second catheter.

19. The method as claimed in any one of claims 16 to 18 further including sampling cerebrospinal fluid at a sub-dural location that is different than the first sub-dural location and measuring the concentration of the curcumin agent in the cerebrospinal fluid to estimate its brain tissue concentration.

20. A method for reducing a human brain dementia relating to the presence of a pathogenic substance in cerebrospinal fluid, comprising:
selecting a human for treatment as a patient that has been diagnosed with dementia;
placing a proximal end of a first catheter, having at least a first lumen, in a first sub-meningeal location within the brain of the patient to establish open communication between the first lumen and cerebrospinal fluid of the patient; and
delivering to the cerebrospinal fluid for an extended period of time a curcumin agent selected from at least one of curcumin, a curcumin hybrid and a curcumin analog to interact with the pathogenic substance to attenuate its effect on the brain, wherein the curcumin agent is released from a reservoir connected to the first catheter and achieves a brain tissue concentration of at least 1 µM.

21. The method as claimed in claim 20 further including sampling cerebrospinal fluid at a sub-dural location that is different than the first sub-dural location and measuring the concentration of the curcumin agent in the cerebrospinal fluid to estimate its brain tissue concentration.

22. A curcumin agent selected from at least one of curcumin, a curcumin hybrid and a curcumin analog for use in a method as claimed in any one of claims 1 to 21.
